Europäisches Patentamt

European Patent Office

Office européen des brevets

(11) Publication number: **0 197 054 B1**

# EUROPEAN PATENT SPECIFICATION

(45) Date of publication of patent specification: **15.07.92**

(51) Int. Cl.⁵: **B32B 27/32**, A61J 1/00, A61L 2/26, B65D 30/08

(21) Application number: **85904588.2**

(22) Date of filing: **26.09.85**

(86) International application number:
**PCT/BE85/00015**

(87) International publication number:
**WO 86/02044 (10.04.86 86/08)**

The file contains technical information submitted
after the application was filed and not included in
this specification

(54) **POLYOLEFIN FILM FOR STEAM STERILIZABLE FLEXIBLE CONTAINERS.**

(30) Priority: **28.09.84 US 655494**
**11.12.84 US 680459**

(43) Date of publication of application:
**15.10.86 Bulletin 86/42**

(45) Publication of the grant of the patent:
**15.07.92 Bulletin 92/29**

(84) Designated Contracting States:
**BE DE FR GB IT NL SE**

(56) References cited:
**BE-A- 662 854**
**GB-A- 1 603 128**
**US-A- 4 022 646**

**See also references of WO8602044**

(73) Proprietor: **BAXTER INTERNATIONAL INC. (a Delaware corporation)**
**One Baxter Parkway**
**Deerfield Illinois 60015(US)**

(72) Inventor: **KERSTEN, Jean**
**259, Chaussée de Tournai**
**B-7931 Villers St. Amand(BE)**
Inventor: **LECOMTE, Leon**
**22, rue des Chapelles**
**B-5840 Rhismes(BE)**

(74) Representative: **De Palmenaer, Roger et al**
**Bureau Vander Haeghen S.A. Rue Colonel Bourg 108 A**
**B-1040 Bruxelles(BE)**

## Description

The present invention relates to a container having an inner and outer surface, said container being made from a film structure comprising at least two polyolefin layers.

Flexible, collapsible liquid containers are utilized in the medical industry for parenteral solutions and the like. Typically, these containers consist of a liquid containing body defined by heat sealed walls. Because these containers are utilized to contain fluids that are introduced intravenously into patients, it is necessary that the containers: are essentially transparent; are flexible; essentially free of extractables; and are sterilizable.

Various materials and structures have been utilized for parenteral solution containers. The materials that have been utilized include polyethylene, polyvinyl chloride, polypropylene, poly(ethylene methyl acrylate), and other copolymers.

BE-A-662 854 describes multilayered films made of low density polyethylene (high pressure) and high density polyethylene (low pressure). According to this document, films made of the lay-up of a low density polyethylene layer and a high density polyethylene layer have many disadvantages : among others, deformation, for example during the welding, due to different expansion coefficients.

Such films would thus not be suitable for making steam sterilizable flexible containers.

For avoiding the disadvantages of the films made of the lay-up of a low density polyethylene layer and a high density polyethylene layer, BE-A-662 854 proposes to make films comprising two outer layers of low density polyethylene and an inner layer of high density polyethylene.

US-A-4,022,646 relates to a process of co-orientation lamination of two ethylene polymer films, the polymer of a first film having a higher melting point (a higher density) than that of the polymer of the second film.

The application of a low density ethylene polymer film to the high density ethylene polymer film is necessary for the heat-sealing of the oriented film and obtaining products having acceptable properties.

US-A-4,022,646 teaches directly contrary to the present invention i.e. that the layer of the film with the highest density polymer must be the thickest layer.

One of the problems of the prior art film is that if these containers are to be steam sterilized, it is necessary that the outer layer of the film has a sufficiently high softening temperature so that it does not substantially deform during sterilization. In the prior art, creating a steam sterilizable outer layer usually resulted in sacrificing some other desired property. For example, to achieve the desired thermal resistance when a polyolefin is utilized, the thickness of the polyolefin has been increased to such an extent that clarity was impaired.

It has also been found that mechanical properties like impact strength decrease with an increase in the softening temperature of the polyolefin structure. Accordingly, if a polyolefin structure with a higher softening temperature is utilized, an increase in the thickness of the polyolefin film structure is required. This increase in thickness results in poor optical properties and a lack of flexibility that results in an increase in the size of the container. Moreover, because the flexible container is created by the inner surfaces of the polyolefin film being heat sealed together, the increase in thickness and/or softening temperature increases the time necessary for effectuating these seals. This in turn increases the duration of the production cycle necessary to create the flexible container, thereby resulting in a less economical flexible container.

Another example of a film structure that has not provided entirely satisfactory results is a polyethylene film structure. Because of polyethylene's susceptability to deformation, lower sterilization temperatures have been utilized for films constructed from polyethylene. The use of lower sterilization temperatures lengthens the total cycle duration for the production of flexible containers from polyethylene. This results in a less economical production cycle and accordingly a more expensive container.

Thus, there is a need for a new polyolefin film structure for creating steam sterilizable, flexible containers that overcomes the problems of the prior art.

The present invention relates to a container having an inner and outer surface, said container being made from a film structure comprising at least two polyolefin layers. The film structure is a coextruded film structure comprising :

a polyolefin layer constructed from a high density polyethylene for defining the outer surface, said high density polyethylene having a density comprised between 0.935 and 0.965 g/cm$^3$ ;

a polyolefin layer constructed from a medium density polyethylene for defining the inner surface, said medium density polyethylene having a density comprised between 0.917 and 0.930 g/cm$^3$ ;

the softening temperature of the high density polyethylene layer being greater than the softening temperature of the medium density polyethylene layer and the medium density polyethylene layer having a greater thickness than the high density polyethylene layer,

so that said container is essentially transparent, flexible, essentially free of extractables and sterilizable at about 121°C without distorsion.

Accordingly, it is an advantage of the present invention to provide a polyolefin film that can be steam sterilized yet provides a flexible container with sufficient clarity, strength, and can be sealed together on its inner edges.

A still further advantage of the present invention is that it provides a method of creating a film structure that substantially reduces the number of gel breach defects that occur during extrusion.

Additional features and advantages are described in, and will be apparent from, the Detailed Description of the Presently Preferred Embodiments and from the drawings.

Figure 1 illustrates a graph of the density of the film versus the softening temperature of the film.

Figure 2 illustrates a graph of the density of the film versus the impact strength of the film.

Figure 3 illustrates a schematic cross sectional view of an embodiment of the film of this invention.

Figure 4 illustrates a cross sectional view of a further embodiment of the film of this invention.

Figure 5 illustrates a container constructed from the film of this invention.

The containers according to the invention have a film structure which is utilized to make steam sterilizable flexible containers capable of containing a liquid to be maintained and accessed under sterile conditions. These containers typically consist of a liquid containment body defined by thermally sealed walls. An example of such a container is the VIAFLEX® container for parenteral solutions marketed by Travenol Laboratories, Inc. of Deerfield, Illinois.

In one embodiment of the container according to the invention, two bonded polyethylene layers are utilized to produce the film structure.

The structure of the desired polyethylene is based on three considerations: the density of the polyethylene; the softening point of the polyethylene; and the relative thickness of the layer of polyethylene. Because the first layer of the polyethylene will be utilized as the outer layer of the bag, this layer must be capable of being steam sterilized without suffering substantial distortion or melting. Moreover, because the second layer is utilized as the inner layer of the bag, and therefore must be sealed to a corresponding layer, it is important that this layer can readily melt and thereby heat seal.

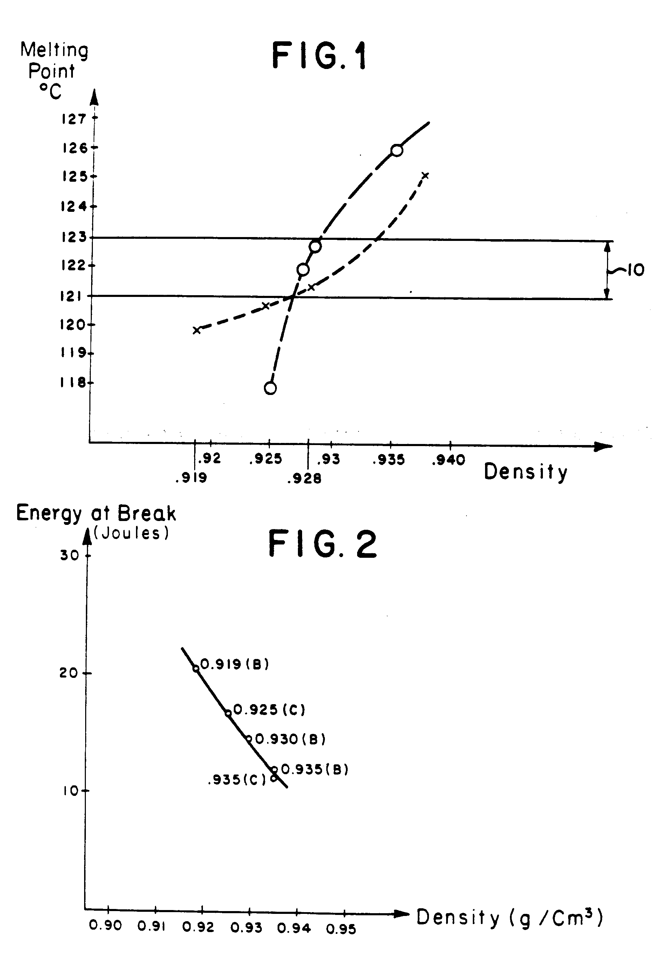

It has been found that the film structure should be chosen based on a function of the density of the polyethylene versus the melting point of polyethylene. Figure 1 illustrates a graph utilizing these parameters. It has been found that if one graphs these parameters, one gets a "window" 10 as shown in Figure 1. It is within this window that the preferred film structures fall. "X" refers to polyethylene comonomers with butene; "0" refers to polyethylene comonomers with octene.

Figure 2 illustrates a graph of the density of polyethylene film versus its impact strength. As illustrated, the impact strength of the film is related to the density of the film. The density of a 150 $\mu$m unsterilized film of polyethylene was tested; the impact strength is measured in Joules. The impact strength was determined by dropping filled containers constructed from the polyethylene film. (B) refers to blown polyethylene film, (C) refers to cast polyethylene film.

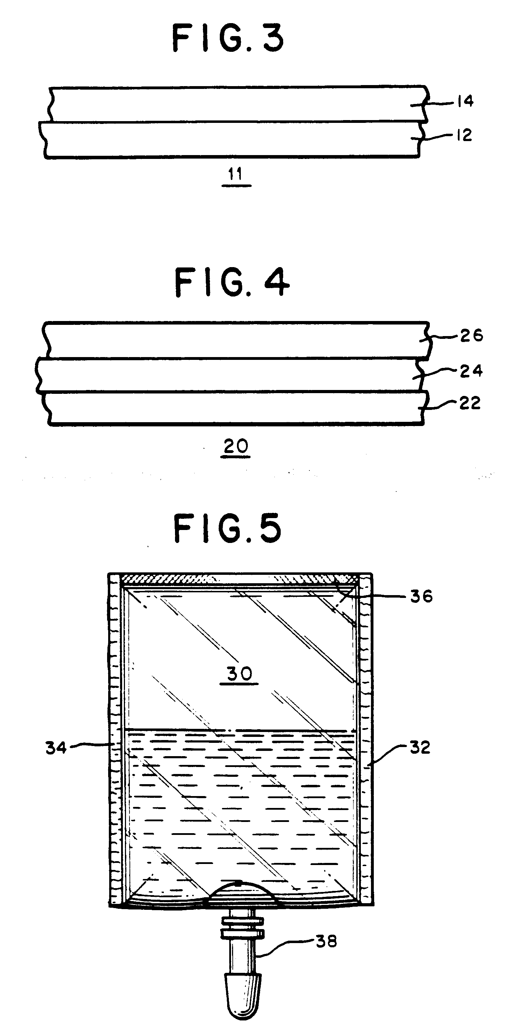

An example of a two layer polyethylene structure that has been found to provide good results as a film for creating a flexible container is as follows. As illustrated in Figure 3, the film 11 comprises a first layer 12 constructed from a high density polyethylene. As used in this disclosure, high density means that the density of the structure is between 0.935 and 0.965 g/cm$^3$. The second layer 14, is bonded to the first layer, and is constructed from medium density polyethylene. As used in this disclosure, medium density means that the density of the structure is between 0.917 and 0.930 g/cm$^3$. Preferably the density of the first polyethylene layer is .95 to .96 g/cm$^3$, and the density of the second layer is .92 to .93 g/cm$^3$. In a most preferred embodiment the density of the first polyethylene layer is about .952 g/cm$^3$ and the density of the second polyethylene layer is about .928 g/cm$^3$. The film of Figure 3 may be made by a conventional coextruded process.

Preferably the linear medium density polyethylene for constructing the film of this invention is a polyethylene copolymer. Preferably, the linear medium density polyethylene contains 2 to 5 weight % of butene or 2 to 5 weight % of octene as copolymer. Because of its greater impact strength polyethylene with 3 weight % octene is preferred. Preferably the polyethylene for constructing the film of this invention is cast polyethylene.

To provide a film that is capable of forming a flexible container that can be steam sterilized, the first polyethylene layer 12 preferably has a softening temperature (Vicat softening point) between about 110°C to 130°C. Preferably, the second polyethylene layer has a softening temperature (Vicat softening point) between about 100°C to 120°C. The resulting multilayer structure provides a film that offers the following advantages over a monolayer polyolefin film: much higher impact strength than monolayer films with an overall higher density; improved optical characteristics than monolayer films with an overall higher density; better surface properties than monolayer films with an overall lower density; superior thermal resistance properties than monolayer films with an overall lower density; and a stronger seal strength than, monolayer films with an overall higher density. Moreover, the flexible container constructed from preferred films of this invention can be steam sterilized at a temperature of to about 121°C without suffering substantial melting or distortion.

Preferably, the thickness of the first polyethylene layer 12 is 20 to 25 μm. The thickness of the second polyethylene layer 14 is preferably 110 to 135 μm. This provides a film construction with the proper balance of properties to provide the advantages set forth above.

In a further preferred embodiment, illustrated in Figure 4, the film structure 20 of the present invention comprises three polyethylene layers. The first polyethylene layer 22 has a greater density and softening point than the second and third polyethylene layers 24 and 26. Preferably the second and third polyethylene layers 24 and 26 have substantially the same density and softening temperature.

It has been found that a three polyethylene film with the following structure provides satisfactory results as a flexible container. The first layer 22 is constructed from a high density polyethylene with a preferred density of .95 to .96 g/cm$^3$. The second layer 24 is constructed from a medium density polyethylene with a preferred density of .92 to .93 g/cm$^3$. The third layer 26 is constructed from a medium density polyethylene with a preferred density of between .92 and .93 g/cm$^3$. Preferably, the softening temperature of the first polyethylene layer 22 is approximately 110°C to 130°C; and the softening temperature of the second and third polyethylene layers 24 and 26 is approximately 100°C to 120°C.

In a most preferred embodiment, the first layer 22 of polyethylene has a density of approximately .952 g/cm$^3$ and the second and third layers of polyethylene have a density of approximately .928 g/cm$^3$.

Preferably the thickness of the first polyethylene layer 22 is 20 to 25 μm and the thickness of the second and third polyethylene layers 24 and 26 is 110 to 135 μm. Preferably the second polyethylene layer 24 has a thickness of 80 to 90 μm and the third polyethylene layer 26 has a thickness of 30 to 50 μm.

The film structure 20 of the three layer film of the present invention provides a film that offers the advantages mentioned above over monolayer polyolefin films. Accordingly, it produces a film structure with good clarity, good surface properties, is steam sterilizable at temperatures of 115°C to 120°C, and provides an inner layer that can be readily heat sealed.

Preferably, the film structure of this invention is manufactured by coextrusion. If a three layer polyethylene structure is utilized, the middle layer may be constructed from a "regrind" material. Because the middle layer is constructed from a regrind material this offers an advantage from a migration standpoint. This is due to the fact that the regrind is not directly in contact with the solution. Moreover, this construction provides a film with increased sealability.

By utilizing a three layer film structure the chances of defects in the film due to a gel breach are substantially lessened. A gel breach is a defect occurring because of unfused particles of resin.

The polyethylene film of this invention may preferably contain the following additives: antioxidants; antiblocking agents; neutralizers (particularly metallic neutralizers such as zinc stearate and calcium stearate); lubricants; stabilizers; and slip agents as required for processing. Preferably the high density polyethylene contains the following additives in the following percentages: a maximum of .15% antioxidant; a maximum of 100 ppm of antiblocking agents or slip agents; a maximum of 1500 ppm neutralizers. Preferably the medium density polyethylene contains the following additives in the following percentages: a maximum of .15% antioxidant; a maximum of 100 ppm of antiblocking agents or slip agents; a maximum of 1500 ppm neutralizers. These additives provide required processing capability to the film structure.

By way of example, and not limitation, an example of the film of this invention will now be set forth:

## Example

The film is a coextrusion having an outer layer, a middle layer, and an inner layer. The middle layer and inner layer are constructed from the same material. The overall film structure comprises: 99.5% by weight linear polyethylene; .15% by weight Irganox 1076; .05% by weight zinc stearate; a maximum of .19% by weight silica; and a maximum of .10% by weight Erucamide®. The zinc stearate acts as a lubricant, the silica acts as an antiblocking agent, and the Erucamide® as a slip agent.

Referring now to the individual layers of the film, the outside layer comprises: 99.84% by weight a polyethylene resin sold under the name Stamylex M 7058® by DSM Belgium S.A., Brussels, Belgium; a maximum of .95% by weight Irganox 1076® sold by Ciba-Geigy; and a maximum of .05% by weight zinc stearate sold by U.C.B. Products Chimiques. The polyethylene resin has a density of approximately .955 g/cm$^3$.

The inner and middle layers comprises: 98% by weight linear medium density polyethylene and a maximum of 1.95% by weight of a masterbatch. The linear medium density polyethylene comprises: 99.84% by weight of a blend of polyethylene resins, the blend of polyethylene having approximately 95-80% by weight Stamylex M 2046® sold by D.S.M. and having a density of approximately .9285 and 5-20% by weight Stamylex M 7058®; a maximum of .95% by weight Irganox 1076® sold by Ciba-Geigy; and a maximum of .05% by weight of zinc stearate sold by U.C.B. Products Chimiques. The masterbatch comprises: 85% by weight linear medium density polyethylene, the polyethylene being the above-stated blend; 10% +% by weight silica sold by Manville de France, under the name Celite 499® and 5% + .5% by weight Erucamide® sold by Croda, France S.A. under the name Eracamide E.R.®.

As illustrated in Figure 5, the film of this invention may be used to produce a container 30 for housing a product to be maintained and accessed under sterile conditions. The container 30 includes side seals 32 and 34, a top seal 36 and a fitment 38. The film of this invention is constructed so that it can be run on a production machine to make a container 30. An example of such a production machine is a form, fill, and seal packaging machine.

## Claims

1. A container having an inner and outer surface, said container being made from a film structure comprising at least two polyolefin layers, characterized in that said film structure is a co-extruded film structure (11,20) comprising :

   a polyolefin layer (14,26) constructed from a high density polyethylene for defining the outer surface, said high density polyethylene having a density comprised between 0.935 and 0.965 g/cm$^3$ ;

   a polyolefin layer (12,22) constructed from a medium density polyethylene for defining the inner surface, said medium density polyethylene having a density comprised between 0.917 and 0.930 g/cm$^3$ ;

   the softening temperature of the high density polyethylene layer (14,26) being greater than the softening temperature of the medium density polyethylene layer (12,22) and the medium density polyethylene layer (12,22) having a greater thickness than the high density polyethylene layer (14,26),

   so that said container is essentially transparent, flexible, essentially free of extractables and sterilizable at about 121°C without distorsion.

2. A sterilizable container according to claim 1, characterized in that the film structure (11,20) comprises between the medium density polyethylene layer (12,22) and the high density polyethylene layer (14,26) a middle layer (24) constructed from a medium density polyethylene, i.e. a polyethylene having a density comprised between 0.917 and 0.930 g/cm$^3$, said middle layer being bonded to the medium density polyethylene layer (22) and to the high density polyethylene layer (26), the combined thickness of the middle layer (24) and of the medium density polyethylene layer (22) being greater than the thickness of the high density polyethylene layer (26).

3. A sterilizable container according to claim 2, characterized in that the high density polyethylene is a polyethylene having a density of 0.95 to 0.96 g/cm$^3$ and the medium density polyethylene is a polyethylene having a density of 0.92 to 0.93 g/cm$^3$.

4. A sterilizable container according to claims 2 and 3, characterized in that the density of the polyethylene of the middle layer (24) and of the medium density polyethylene layer (22) is about the same.

5. A sterilizable container according to claim 2, characterized in that the thickness of the high density polyethylene layer (14,26) is 20 to 25 μm and the thickness of the medium density polyethylene layer(s) (12,22,24) is 110 to 135 μm.

6. A sterilizable container according to claims 2 and 5, characterized in that the middle layer (24) has a thickness of 80 to 90 μm and the medium density polyethylene layer (22) has a thickness of 30 to 50 μm.

7. A sterilizable container according to claims 2 and 3, characterized in that the film is produced by co-extrusion and the middle layer is constructed from a regrind material.

8. A sterilizable container according to claims 1 and 3, characterized in that the high density polyethylene layer and the medium density polyethylene layer include an antioxidant, antiblocking agent and stabilizer.

9. Film structure (11,20) for making a sterilizable container (30) according to anyone of the preceding claims.

## Revendications

1. Conteneur présentant une surface interne et une surface externe, ledit conteneur étant réalisé au moyen d'une structure en film comprenant au moins deux couches de polyoléfines, caractérisé en ce que ladite structure en film est une structure en film coextrudée (11, 20) comprenant :

- une couche de polyoléfine (14, 26) réalisée au moyen d'un polyéthylène haute densité pour définir la surface externe, ledit polyéthylène haute densité ayant une densité comprise entre 0,935 et 0,965 g/cm$^3$ ;
- une couche de polyoléfine (12, 22) réalisée au moyen d'un polyéthylène moyenne densité, ledit polyéthylène moyenne densité ayant une densité comprise entre 0,917 et 0,930 g/cm$^3$,

la température de ramollissement de la couche en polyéthylène haute densité (14, 26) étant supérieure à la témpérature de ramollissement de la couche en polyéthylène moyenne densité (12, 22) et la couche en polyéthylène moyenne densité (12, 22) ayant une plus grosse épaisseur que la couche en polyéthylène haute densité (14, 26), de sorte que le conteneur soit essentiellement transparent, flexible, essentiellement exempt de particules extractables et stérilisable à environ 121°C sans subir de déformation.

2. Un conteneur stérilisable suivant la revendication 1, caractérisé en ce que la structure (11, 20) du film comprend entre la couche de polyéthylène moyenne densité (12, 22) et la couche en polyéthylène haute densité (14, 26) une couche intermédiaire (24) réalisée en polyéthylène moyenne densité, c'est-à-dire un polyéthylène ayant une densité comprise entre 0,917 et 0,930 g/cm$^3$, ladite couche intermédiaire étant reliée à la couche en polyéthylène moyenne densité (22) et à la couche en polyéthylène haute densité (26), l'épaisseur combinée de la couche intermédiaire (24) et de la couche en polyéthylène moyenne densité (22) étant supérieure à l'épaisseur de la couche en polyéthylène haute densité (26).

3. Un conteneur stérilisable selon la revendication 2, caractérisé en ce que le polyéthylène haute densité est un polyéthylène ayant une densité de 0,95 à 0,96 g/cm$^3$ et le polyéthylène de moyenne densité est un polyéthylène ayant une densité de 0,92 à 0,93 g/cm$^3$.

4. Un conteneur stérilisable selon les revendications 2 et 3, caractérisé en ce que la densité du polyéthylène de la couche intermédiaire (24) et de la couche en polyéthylène moyenne densité (22) est sensiblement la même.

5. Un conteneur stérilisable selon la revendication 2, caractérise en ce que l'épaisseur de la couche en polyéthylène haute densité (14, 26) est de 20 à 25 $\mu$m et l'épaisseur de la ou des couches en polyéthylène moyenne densité est de 110 à 135 $\mu$m.

6. Un conteneur stérilisable selon les revendications 2 et 5, caractérisé en ce que la couche intermédiaire (24) a une épaisseur de 80 à 90 $\mu$m et la couche en polyéthylène moyenne densité (22) a une épaisseur de 30 à 50 $\mu$m.

7. Un conteneur stérilisable selon les revendications 2 et 3, caractérisé en ce que le film est obtenu par co-extrusion et la couche intermédiaire est réalisée au moyen d'une matière rebroyée.

8. Un conteneur stérilisable selon les revendications 1 et 3, caractérisé en ce que la couche en polyéthyléne haute densité et la couche en polyéthylène moyenne densité comprennent un antioxyant, un agent d'antiadhérence et un stabilisant.

9. Structure en film (11, 20) pour réaliser un conteneur stérilisable (30) suivant l'une quelconque des revendications précédentes.

**Patentansprüche**

1. Behälter mit einer inneren und einer äußeren Oberfläche, wobei der Behälter hergestellt ist aus einer Folienstruktur, umfassend mindestens zwei Polyolefinschichten, dadurch gekennzeichnet, daß die Folienstruktur eine coextrudierte Folienstruktur (Verbundfolie) (11,20) ist, umfassend:

eine Polyolefinschicht (14,26), bestehend aus einem Polyethylen hoher Dichte, die die Außenseite bilden soll, wobei das Polyethylen hoher Dichte eine Dichte zwischen 0,935 und 0,965 g/cm$^3$ aufweist;

eine Polyolefinschicht (12,22), bestehend aus einem Polyethylen mittlerer Dichte, die die Innenseite bilden soll, wobei das Polyethylen mittlerer Dichte eine Dichte zwischen 0,917 und 0,930 g/cm$^3$ besitzt;

wobei die Erweichungstemperatur der Schicht aus Polyethylen hoher Dichte (14,26) größer ist als die Erweichungstemperatur der Schicht aus Polyethylen mittlerer Dichte (12,22) und die Schicht aus Polyethylen mittlerer Dichte (12,22) eine größere Dicke besitzt als die Schicht aus Polyethylen hoher Dichte (14,26), so daß der Behälter im wesentlichen transparent, flexibel, im wesentlichen frei von extrahierbaren Bestandteilen und bei etwa 121°C ohne Zerstörung sterilisierbar ist.

2.  Sterilisierbarer Behälter nach Anspruch 1, dadurch gekennzeichnet, daß die Folienstruktur (11,20) zwischen der Schicht aus Polyethylen mittlerer Dichte (12,22) und der Schicht aus Polyethylen hoher Dichte (14,26) eine mittlere Schicht (24) umfaßt, bestehend aus einem Polyethylen mittlerer Dichte, d.h. einem Polyethylen mit einer Dichte zwischen 0,917 und 0,930 g/cm$^3$, wobei diese mittlere Schicht an die Schicht aus Polyethylen mittlerer Dichte (22) und die Schicht aus Polyethylen hoher Dichte (26) gebunden ist, wobei die gesamte Dicke der mittleren Schicht (24) und der Schicht aus Polyethylen mittlerer Dichte (22) größer ist als die Dicke der Schicht aus Polyethylen hoher Dichte (26).

3.  Sterilisierbarer Behälter nach Anspruch 2, dadurch gekennzeichnet, daß das Polyethylen hoher Dichte ein Polyethylen mit einer Dichte von 0,95 bis 0,96 g/cm$^3$ und das Polyethylen mittlerer Dichte ein Polyethylen mit einer Dichte von 0,92 bis 0,93 g/cm$^3$ ist.

4.  Sterilisierbarer Behälter nach den Ansprüchen 2 und 3, dadurch gekennzeichnet, daß die Dichte des Polyethylens der mittleren Schicht (24) und der Schicht aus Polyethylen mittlerer Dichte (22) etwa gleich sind.

5.  Sterilisierbarer Behälter nach Anspruch 2, dadurch gekennzeichnet, daß die Dicke der Schicht aus Polyethylen hoher Dichte (14,26) 20 bis 25 μm und die Dicke der Schicht(en) aus Polyethylen mittlerer Dichte (12,22,24) 110 bis 135 μm beträgt.

6.  Sterilisierbarer Behälter nach den Ansprüchen 2 und 5, dadurch gekennzeichnet, daß die mittlere Schicht (24) eine Dicke von 80 bis 90 μm und die Schicht aus Polyethylen mittlerer Dichte (22) eine Dicke von 30 bis 50 μm aufweist.

7.  Sterilisierbarer Behälter nach den Ansprüchen 2 und 3, dadurch gekennzeichnet, daß die Folie hergestellt worden ist durch Coextrusion und die mittlere Schicht aus wieder vermahlenem Material besteht.

8.  Sterilisierbarer Behälter nach den Ansprüchen 1 und 3, dadurch gekennzeichnet, daß die Schicht aus Polyethylen hoher Dichte und die Schicht aus Polyethylen mittlerer Dichte ein Antioxidans, Antiblockmittel und Stabilisator enthalten.

9.  Folienstruktur (11,20) zur Herstellung eines sterilisierbaren Behälters (30) nach einem der vorangehenden Ansprüche.

FIG.1

FIG. 2

# FIG. 3

# FIG. 4

# FIG. 5